# EUROPEAN PATENT APPLICATION

(11) **EP 4 134 240 A1**
(43) Date of publication of application: **15.02.2023**
(21) Application number: 21785405.8
(22) Date of filing: 05.04.2021
(51) Int. Cl.: B41J 2/01, C09D 11/328, A61K 9/44, B41M 5/00, A61K 47/14, A61K 47/22

(54) **EDIBLE AQUEOUS INKJET INK AND TABLET**

(30) Priority: 09.04.2020 JP 2020070591
(71) Applicant: TOPPAN INC., Tokyo 110-0016 (JP)
(72) Inventor: KOSEKI Seiya, Tokyo 110-0016 (JP); FUKUSHIRO Masato, Tokyo 110-0016 (JP); ISHIKAWA Hideki, Tokyo 110-0016 (JP); HOSHINO Yuichi, Tokyo 110-0016 (JP); SAITO Masatoshi, Tokyo 110-0016 (JP)
(74) Representative: TBK
(86) International application number: PCT/JP2021/014546
(87) International publication number: WO 2021/206067

(57) **Abstract**

The present invention aims to provide an edible aqueous inkjet ink and a tablet. The edible aqueous inkjet ink contains a relatively large amount of volatile alcohol, which has excellent transfer durability and reduces ink ejection failure. The tablet includes a portion printed with the edible aqueous inkjet ink. The edible aqueous inkjet ink according to the present embodiment includes food coloring, ethanol, and a surfactant which has a hydrophilic-lipophilic balance value (HLB) in a range of 16 or more and 20 or less. The food coloring may preferably be sodium copper chlorophyllin. The surfactant may preferably be a sucrose fatty acid ester.

## Description

### [Technical Field]

The present invention generally relates to an edible aqueous inkjet ink and a tablet.

### [Background Art]

There are some edible inks for inkjet printing (hereinafter referred to simply as an "inkjet ink"). PTL 1 discloses techniques related to such edible inkjet inks.

There are some inkjet inks which are water-based inkjet inks that contain a large amount of volatile alcohol (hereinafter also referred to as "high-alcohol inkjet ink"). For example, some high-alcohol inkjet inks contain about 40 mass% of volatile alcohol relative to the total mass of the ink.

Although high-alcohol inkjet inks can suppress transfer (so-called color transfer) of ink when they come into contact with a printed tablet, many of them have excellent transfer durability. However, when a high-alcohol inkjet ink is stored for a long time or continuous printing is performed using the inkjet ink, ink ejection failure may occur during printing due to thickening of the ink caused by volatilization of solvent such as alcohol contained in the ink or due to deposition of a dye due to the volatilization of the solvent. In other words, some high-alcohol inkjet inks in the conventional technology have excellent transfer durability but have low (insufficient) ejection stability during printing.

### [Citation List]

### [Patent Literature]

[PTL 1] JP 2006-169301 A

### [Summary of the Invention]

### [Technical Problem]

The present invention has been made in view of the above circumstances, and aims to provide an edible aqueous inkjet ink and a tablet. The edible aqueous inkjet ink has excellent transfer durability due to its relatively high content of volatile alcohols, and also reduces ink ejection failure. The tablet includes a portion printed with the edible aqueous inkjet ink.

### [Solution to Problem]

An edible aqueous inkjet ink according to one aspect of the present invention includes a dye, ethanol, and a surfactant which has a hydrophilic-lipophilic balance (HLB) value in a range of 16 or more and 20 or less.

Further, an edible aqueous inkjet ink according to one aspect of the present invention includes a dye, ethanol, and sucrose fatty acid ester.

### [Advantageous Effects of the Invention]

The edible aqueous inkjet ink according to one aspect of the present embodiment has excellent transfer durability because it contains a relatively large amount of volatile alcohol, and also reduces ink ejection failure. The edible aqueous inkjet ink according to one aspect of the present invention achieves higher ejection stability than conventional inkjet inks while maintaining the same level of excellent transfer durability as conventional inkjet inks.

### [Brief Description of the Drawings]

Fig. 1 is a schematic cross-sectional view illustrating an example of a tablet (uncoated tablet) according to an embodiment of the present invention.
Fig. 2 is a schematic cross-sectional view illustrating an example of a tablet (film-coated tablet) according to an embodiment of the present invention.
Fig. 3 is a diagram illustrating an example of a printed image for a tablet (uncoated tablet) according to an embodiment of the present invention.
Fig. 4 is a diagram illustrating an example of a printed image for a tablet (film-coated tablet) according to an embodiment of the present invention.
Fig. 5 is a set of diagrams illustrating evaluation criteria for evaluating ejection stability (continuous printability) in examples of the present invention.
Fig. 6 is a set of diagrams illustrating evaluation criteria for evaluating ejection stability (intermittent resumability) in examples of the present invention.

### [Description of the Embodiments]

An inkjet ink according to an embodiment of the present invention is an inkjet ink which is used, for example, for printing characters, an image, or the like on a surface of a medical tablet by an inkjet printing method. The following description specifically explains an inkjet ink according to an embodiment of the present invention and a tablet having a portion printed with the inkjet ink.

### [Composition of inkjet ink]

The inkjet ink according to the present embodiment contains at least food coloring (food dye), a dispersion medium (solvent) capable of dispersing the food coloring, and a surfactant. The food coloring according to the present embodiment may be, for example, sodium copper chlorophyllin, Blue No. 1, Blue No. 2, Red No. 102, Red No. 2, Red No. 3, Yellow No. 4, or Yellow No. 5. The dispersion medium according to the present embodiment also serves as a drying accelerator and is ethanol. The surfactant according to the present embodiment has an HLB value in the range of 16 or more and 20 or less, and the example of the surfactant may be sucrose fatty acid ester (DK ester SS manufactured by DKS Co. Ltd.). Such a configuration minimizes the volatilization of the solvent even when inkjet ink is stored for a long period of time without being used or continuous printing is performed using the inkjet ink, and thus maintains the stability in ejecting ink (printing stability), which leads to ensuring printing resumability (intermittent resumability), and continuous printability. If the HLB value of the surfactant is less than 16, sometimes the volatilization of the solvent may not be sufficiently suppressed, and thus the inkjet ink tends to dry out, leading to the deterioration of the ejection stability. HLB is an acronym for Hydrophilic-Lipophilic Balance with a maximum value of 20.

Advantageous effects that can be obtained by the inkjet ink of the present embodiment will be described.

In the past, in the technical field to which the inkjet ink according to the present embodiment belongs, there had been a method of using decaglyceryl monostearate as an ejection stabilizer (surfactant) to be added to ink to improve the ejection stability of inkjet ink.

On the other hand, in the inkjet ink according to the present embodiment, by selecting the type and physical properties of the surfactant to be added to the ink, the volatilization of the solvent is suppressed, and by reducing the thickening of the ink and the deposition of the dye due to the volatilization of the solvent, the occurrence of ink ejection failure during printing is reduced. More specifically, for example, the inventors have found that the use of a surfactant having a relatively high hydrophilic-lipophilic balance (HLB) as a surfactant to be added to the ink has an effect of suppressing the volatilization of the solvent on the inkjet nozzle surface. Therefore, the inventors have realized that by adjusting the HLB value of the surfactant to be included in the ink or by selecting the type of the surfactant to be included in the ink, the volatilization of the solvent is minimized even when the inkjet ink is stored for a long period of time without being used or continuous printing is performed using the inkjet ink, and thus stability in ejecting ink is maintained, which leads to ensuring printing restartability (intermittent resumability) and continuous printability.

The components constituting the inkjet ink according to the present embodiment will be described below.

### (Surfactant)

The surfactant contained in the inkjet ink according to the present embodiment has an HLB value in the range of 16 or more and 20 or less, and the example of the surfactant may be sucrose fatty acid ester (DK ester SS manufactured by DKS Co. Ltd.). The surfactant having the above components enables a monomolecular film of molecules comprising the surfactant to be formed on the surface of the head nozzle of the inkjet printer, thereby suppressing the volatilization of ethanol used as a drying accelerator. This makes it possible to reduce thickening of ink and deposition of dyes due to the volatilization of ethanol, thereby reducing ink ejection failure during printing, even when inkjet ink is stored for a long time or continuous printing is performed using the inkjet ink. In addition, since ethanol volatilizes quickly after printing, transfer durability is obtained.

The HLB value of the surfactant contained in the inkjet ink according to the present embodiment is preferably in the range of 18 or more and 20 or less, and more preferably in the range of 19 or more and 20 or less. The inkjet ink having the above configuration makes it possible to further suppress volatilization of the above solvent and further reduce thickening of ink and deposition of dyes due to the volatilization of the above solvent, thereby reducing ink ejection failure during printing.

The inkjet ink according to the present embodiment may be in a form containing only one kind of surfactant or may be in a form containing two or more kinds of surfactant. In detail, the inkjet ink according to the present embodiment may be in a form containing only a surfactant (sucrose fatty acid ester) having an HLB value in the range of 16 or more and 20 or less, or may be a form containing other surfactants together with a surfactant (sucrose fatty acid ester) having an HLB value in the range of 16 or more and 20 or less. The inkjet ink having the above configuration makes it possible to further suppress volatilization of the above solvent and further reduce thickening of ink and deposition of dyes due to the volatilization of the above solvent, thereby reducing ink ejection failure during printing.

The surfactant contained in the inkjet ink according to the present embodiment may include a first surfactant having a relatively high HLB value and a second surfactant having a relatively low HLB value (a surfactant having a lower HLB value than the first surfactant). The inkjet ink having the above configuration makes it possible to further suppress volatilization of the above solvent and further reduce thickening of ink and deposition of dyes due to the volatilization of the above solvent, thereby reducing ink ejection failure during printing.

When two or more surfactants are used as surfactants contained in the inkjet ink of the present embodiment, the content of the first surfactant having a relatively high HLB value may be higher than the content of the second surfactant having a relatively low HLB value (a surfactant having a lower HLB value than the first surfactant). The inkjet ink having the above configuration makes it possible to further suppress volatilization of the above solvent and further reduce thickening of ink and deposition of dyes due to the volatilization of the above solvent, thereby reducing ink ejection failure during printing. Specifically, the content of the first surfactant having a relatively high HLB value may be in the range of 1.1 times or more and 2 times or less the content of the second surfactant having a relatively low HLB value (a surfactant having a lower HLB value than the first surfactant).

The inkjet ink of the present embodiment needs to contain a surfactant whose HLB value is in the range of 16 or more and 20 or less, and the HLB value of other surfactants may be less than 16 if the content of surfactants whose HLB value is in the range of 16 or more and 20 or less is greater than that of other surfactants. Even with the above configuration, the volatilization of the above solvent is further suppressed and the thickening of ink and deposition of dyes due to the volatilization of the above solvent are further reduced, thereby reducing ink ejection failure during printing.

The fatty acid portion of the sucrose fatty acid esters may be a short-chain fatty acid (lower fatty acid) having 2 to 4 carbon atoms, a medium-chain fatty acid having 5 to 12 carbon atoms, or a long-chain fatty acid (higher fatty acid) having 13 or more carbon atoms. Among these, a sucrose fatty acid ester containing a medium-chain fatty acid having 5 to 12 carbon atoms is more preferred. The sucrose fatty acid ester containing a medium-chain fatty acid having 8 to 10 carbon atoms is most preferred. The inkjet ink having the above configuration makes it possible to further suppress volatilization of the above solvent and further reduce thickening of ink and deposition of dyes due to the volatilization of the above solvent, thereby reducing ink ejection failure during printing.

In the inkjet ink according to the present embodiment, the surfactant may have a content in the range of 0.5 mass% or more and 5 mass% or less of the total amount of the inkjet ink, and more preferably in the range of 1 mass% or more and 3 mass% or less of the total amount of the inkjet ink. Such a configuration reliably forms a monomolecular film of molecules constituting the surfactant on the surface of the head nozzle of the inkjet printer, thereby suppressing the volatilization of ethanol used as a drying accelerator. Therefore, thickening of ink and deposition of dyes due to the volatilization of ethanol can be further reduced, and even when inkjet ink is stored for a long time or continuous printing is performed using the inkjet ink, ink ejection failure during printing can be further reduced.

### (Ethanol)

The solvent (drying accelerator) contained in the inkjet ink according to the present embodiment is ethanol. When the solvent is ethanol, the volatility is high enough to make the inkjet ink having excellent transfer durability.

In the inkjet ink according to the present embodiment, the ethanol may have a content in the range of 12 mass% or more and 55 mass% or less of the total amount of the inkjet ink, and more preferably in the range of 25 mass% or more and 50 mass% or less of the total amount of the inkjet ink. Such a configuration imparts higher ejection stability than that of the conventional technologies while reliably maintaining excellent transfer durability.

The inkjet ink according to the present embodiment may be added with solvents other than ethanol as described above. Examples of the solvent which can be added to the inkjet ink according to the present embodiment include purified water, glycerin, propylene glycol, polyethylene glycol 300 (average molecular weight 300), 1-propanol, 2-propanol, and ethyl lactate. There is no particular limitation on the blending ratio but the ink preferably contains any of glycerin, propylene glycol, or polyethylene glycol 300 in the range of 1 wt% or more and 30 wt% or less to prevent the ink from drying in the nozzle.

As mentioned above, the solvent (drying accelerator) contained in the inkjet ink according to the present embodiment is preferably composed only of ethanol, but a solvent other than ethanol may be added. When ethanol and a solvent other than ethanol are used as the solvent contained in the inkjet ink according to the present embodiment, it is preferable that the content of the ethanol is larger than that of the solvent other than ethanol. For example, the content of ethanol may be in the range of 1.5 times or more and 5 times or less the content of a solvent other than ethanol.

### (Food coloring)

The food coloring contained in the inkjet ink according to the present embodiment may be, for example, sodium copper chlorophyllin (Chlorophyllin copper complex sodium, CAS Number: 28302-36-5), Blue No. 1, Blue No. 2, Red No. 102, Red No. 2, Red No. 3, Yellow No. 4, or Yellow No. 5. When the food coloring is the above dye, it is possible to obtain higher ejection stability than that of the conventional technologies while reliably maintaining excellent transfer durability. Blue No. 1 Colorant (FDA name: FD & C Blue No. 1, Color index name: Food Blue 2, CAS number: 3844-45-9) is a colorant also known as Brilliant Blue FCF. Blue No. 2 Colorant (FDA Name: FD & C Blue No.2, Color Index Name: Acid Blue 74, CAS Number: 860-22-0) is a colorant also known as Indigo Carmine. Red No. 102 Colorant (Color Index Name: Acid Red 18, CAS Number: 2611-82-7) is a colorant also known as New Coccine. Red No. 2 Colorant (FDA Name: FD & C Red No.2, Color Index Name: Acid Red 27, CAS Number: 915-67-3) is a colorant also known as Amaranth. Red No. 3 Colorant (FDAName: FD & C Red No.3, Color Index Name: Acid Red 51, CAS Number: 16423-68-0) is a colorant also known as Erythrosine. Yellow No. 4 Colorant (FDA Name: FD & C Yellow No.5, Color Index Name: Acid Yellow 23, CAS Number: 1934-21-0) is a colorant also known as Tartrazine. Yellow No. 5 Colorant (FDA Name: FD & C Yellow No.6, Color Index Name: Food Yellow 3, CAS Number: 2783-94-0) is a colorant also known as Sunset Yellow FCF.

The inkjet ink according to the present embodiment may contain, as food coloring, for example, a single pigment or a mixture of a plurality of pigments.

In the inkjet ink according to the present embodiment, the food coloring may have a content in the range of 2.5 mass% or more and 10 mass% or less of the total amount of the inkjet ink, and more preferably in the range of 3 mass% or more and 7.5 mass% or less of the total amount of the inkjet ink. Such a configuration maintains the readability (visibility) of printed text, and also realizes excellent light resistance which will be described below.

The "Color Index Name" mentioned above is determined by the American Association of Textile Chemists and Colorists. The "FDAName" mentioned above is determined by the U.S. FDA (U.S. Food and Drug Administration). In the present embodiment, each available colorant (each substance) is specified using CAS Number, but the present invention is not limited to this. As a matter of course, for example, pigments that can be used in the present embodiment include those pigments which have the same names as those of the pigments (substances) mentioned in the present embodiment but have different CAS Numbers because of being geometric isomers, stereoisomers, materials comprising isotopes, or salts thereof. If an isomer or the like is not present or an available colorant (substance) is specified (limited), the substance (compound) with the CAS number described in the present embodiment can be used.

As a food coloring which can be added to the inkjet ink of the present embodiment, even a pigment other than the above-described pigment can be used without any particular limitation as long as it is edible.

Pigments that can be added to the inkjet ink of the present embodiment can be appropriately selected from, for example, conventionally known synthetic and natural food coloring.

Examples of the synthetic food coloring include a tar colorant, a natural colorant derivative, and a natural synthetic colorant. Examples of the tar colorants include Food Red No. 40 (Allura Red AC, FDA Name: FD & C Red No.40, Color Index Name: Food Red 17, CAS Number: 25956-17-6), Food Red No. 104 (Phloxine, FDA Name: D & C Red No.28, Color Index Name: Acid Red 92, CAS Number: 18472-87-7), Food Red No. 105 (Rose Bengal, Color Index Name: Acid Red 94, CAS Number: 632-69-9), Food Red No. 106 (Acid Red, Color Index Name: Acid Red 52, CAS Number: 3520-42-1), Food Red No. 2 Aluminum Lake (FD & C Red No.2 Aluminum Lake), Food Red No. 3 Aluminum Lake (FD & C Red No.3 Aluminum Lake), Food Red No. 40 Aluminum Lake (FD & C Red No.40 Aluminum Lake), Food Yellow No. 4 Aluminum Lake (FD & C Yellow No.5 Aluminum Lake), Food Yellow No. 5 Aluminum Lake (FD & C Yellow No.6 Aluminum Lake), Food Blue No. 1 Aluminum Lake (FD & C Blue No.1 Aluminum Lake), and Food Blue No. 2 Aluminum Lake (FD & C Blue No.2 Aluminum Lake). Examples of the natural colorant derivative include norbixin potassium or the like. Examples of the natural synthetic colorant include β-carotene, riboflavin, or the like.

Furthermore, examples of the natural food colorings include anthocyanin pigments, carotenoid pigments, quinone pigments, flavonoid pigments, betaine pigments, Monascus pigments, and other pigments originating from natural products. Examples of the anthocyanin colorant include red radish colorant, red cabbage colorant, red rice colorant, elderberry colorant, cowberry colorant, gooseberry colorant, cranberry colorant, salmon berry colorant, perilla colorant, sim blueberry colorant, strawberry colorant, dark sweet cherry colorant, cherry colorant, hibiscus colorant, huckleberry colorant, grape juice colorant, grape skin colorant, black currant colorant, blackberry colorant, blueberry colorant, plum colorant, whortleberry colorant, boysenberry colorant, mulberry colorant, purple sweet potato colorant, purple corn colorant, Chinese purple potato colorant, raspberry colorant, red currant colorant, loganberry colorant, and other anthocyanin colorants. Examples of the carotenoid colorant include annatto pigment, gardenia yellow pigment, and other carotenoid pigments. Examples of the quinone colorant include cochineal colorant, Lithospermi radix colorant, lac colorant, and other quinone colorants. Examples of the flavonoid colorant include safflower yellow colorant, kaoliang colorant, onion colorant, and other flavonoid colorants. Examples of the betaine colorant include beet red colorant. Examples of the Monascus colorant include Monascus red colorant and Monascus yellow colorant. Examples of other colorants originating from natural products include turmeric colorant, clerodendrum trichotomum colorant, gardenia red colorant, and spirulina blue colorant.

The hue of the inkjet ink can be adjusted by the combination of the above pigments or by the combination of the contents of the pigments. For example, if the hue of the inkjet ink is desired to be gray, a green pigment of sodium copper chlorophyllin, a red pigment of New Coccine (Food Red No. 102) or erythrosine (Food Red No. 3), and a blue pigment of Brilliant Blue FCF (Food Blue No. 1) may be selected as food coloring. Also, if the hue of the inkjet ink is desired to be yellow-green, sodium copper chlorophyllin which is a green pigment, and tartrazine (Food Yellow No. 4) which is a yellow pigment may be selected as food coloring.

### (Internal-sizing resin)

The inkjet ink according to the present embodiment may contain an internal-sizing resin in addition to the above pigments or solvents. The additive resin which can be added to the inkjet ink according to the present embodiment may be a resin-like substance which is edible, water-soluble powder, paste or flake, and can form a film on the tablet surface by drying after printing. Examples of the internal-sizing resin include polyvinyl alcohol (PVA), hydroxypropyl cellulose (HPC), hydroxypropyl methylcellulose (HPMC), polyvinylpyrrolidone (PVP), high molecular weight polyethylene glycol (PEG) such as polyethylene glycol 4000 or polyethylene glycol 1540, shellac resin, methacrylic acid copolymer (product name: Eudragit S100), maltodextrin, and erythritol.

### (Leveling agent)

The inkjet ink according to the present embodiment may contain a leveling agent in addition to the above pigments, solvents, or internal-sizing resins. The levelling agent that can be added to the inkjet ink according to the present embodiment may be an edible and water-soluble surfactant. Examples of the levelling agent include polyglycerin fatty acid ester (e.g., Decaglyceryl distearate Q-182S or Decaglyceryl monolaurate Q-12S manufactured by Taiyo Kagaku Co., Ltd.), sorbitan fatty acid ester (e.g., NIKKOLSL-10 manufactured by Nikko Chemicals Co., Ltd.), sucrose fatty acid ester (e.g., DK Ester F-110 manufactured by DKS Co. Ltd.), and polysorbate (Emazole S-120 series manufactured by Kao Corporation).

### [Printing method]

The printing method using the inkjet ink according to the present embodiment is not particularly limited. Printing using an inkjet device, such as a commercially available inkjet printer, is possible. Therefore, the inkjet ink according to the present embodiment has a wide range of application and thus is very useful. For example, the inkjet ink according to the present embodiment can be printed with a drop-on-demand inkjet device including a piezoelectric element (piezoelectric ceramic) as an actuator, or with other types of inkjet devices.

Examples of the drop-on-demand inkjet device include a thermal inkjet device that ejects inkjet ink with a water vapor pressure generated by instantaneously heating micro-heating elements to a high temperature (200 to 300°C), an electrostatic device that ejects inkjet ink by electrostatically vibrating an actuator, and an ultrasonic wave device that uses a cavitation phenomenon caused by ultrasonic waves. If the inkjet ink of the present embodiment has charging performance, a continuous injection device may be used.

### [Tablet]

The inkjet ink of the present embodiment may be used for printing characters or an image, for example, on the surface of a tablet using the above printing methods. The following description explains a configuration of a tablet having an image printed by the inkjet ink according to the present embodiment.

The tablet of the present embodiment is, for example, a medical tablet. The "medical tablet" includes, for example, a film-coated tablet having a water-soluble surface layer formed on the outermost surface thereof as well as an uncoated tablet (naked tablet), a sugar-coated tablet, an enteric tablet, an orally disintegrating tablet and the like.

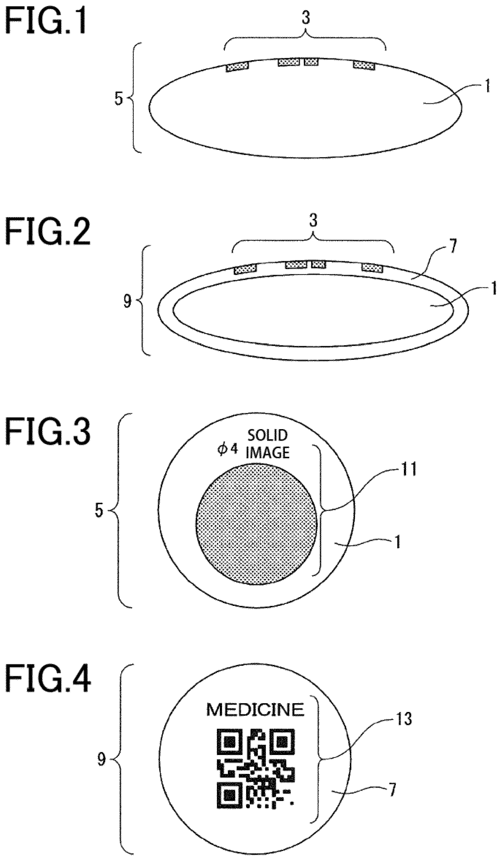

Fig. 1 is a schematic cross-sectional view illustrating an example of a medical tablet (uncoated tablet) on which characters or an image is printed. Fig. 1 shows a cross section of an uncoated tablet 5 in which a printed image 3 such as of characters is printed on top face of a tablet body 1

Fig. 2 is a schematic cross-sectional view illustrating an example of a medical tablet (film-coated (FC) tablet) having a print pattern (characters or image) Fig. 2 shows a cross section of a film-coated tablet 9 in which a printed image 3 such as of characters is printed on top face of a tablet 1 which is covered with a film coating layer 7.

As shown in Fig. 3, in the present embodiment, a solid image may be printed on an uncoated tablet as a printed image 11, or as shown in Fig. 4, a two-dimensional barcode may be printed on a film coated tablet as a printed image on film coated tablet 13.

There is no particular limitation on the active ingredients contained in the medical tablet. Examples of the active ingredients include a substance effective for preventing or treating various diseases (e.g., a substance having sleep-inducing effect, an tranquilizer activity, an antibacterial activity, an antihypertensive effect, an anti-angina activity, an analgesic effect, an antiinflammatory activity, a tranquilizing effect, a diabetes treatment activity, a diuretic effect, an anticholinergic activity, an anti-hyperacidity effect, an antiepileptic effect, an ACE inhibitory activity, a β-receptor antagonist or agonist activity, an anesthetic action, an appetite suppressant action, an antiarrhythmic effect, an antidepressant effect, an anticoagulant activity, an antidiarrheal effect, an antihistamine activity, an antimalarial effect, an antitumor activity, an immunosuppressive activity, an antiparkinsonian effect, an antipsychotic effect, an antiplatelet activity, an antihyperlipidemic effect, and the like), a substance having a scavenging effect, and a substance having a scent or a deodorant action, but are not limited thereto.

The tablet according to the present embodiment may as necessary contain a carrier that is acceptable for its application, together with an active ingredient. For example, medical tablets may comprise carriers which are tolerated from pharmaceutical perspectives. As carriers tolerated from pharmaceutical perspectives, various organic or inorganic carrier materials may be mixed, which are commonly used as pharmaceutical materials, and an appropriate amount of excipients, lubricants, binders, disintegrating agents, thickeners or the like may be mixed thereto as appropriate. As necessary, additives, such as an antiseptic, antioxidant, colorant or sweetener, may be used.

While the present embodiment has been described taking an example of a medical tablet as a tablet, it should be appreciated that the present invention is not limited to thereto. There is no particular limitation on what the inkjet ink according to the present embodiment is printed on. The ink may be printed on the surface of various tablets, such as a tablet to be administered to animals other than humans (e.g., pets, domestic animals, poultry), a feed tablet, fertilizer tablet, a cleaning agent tablet, a lemonade tablet, or the like. The inkjet ink of the present embodiment does not particularly limit the size of an object to be printed, but may be applied to tablets of various sizes.

### (Effects of the present embodiment)

(1) The inkjet ink according to the present embodiment includes food coloring, ethanol, and a surfactant which has a hydrophilic-lipophilic balance (HLB) value in the range of 16 or more and 20 or less.

Such a configuration reduces ink ejection failure while enabling excellent transfer durability compared to conventional technologies.

(2) The inkjet ink according to the present embodiment includes food coloring, ethanol, and sucrose fatty acid ester.

Such a configuration reduces ink ejection failure while enabling excellent transfer durability compared to conventional technologies.

(3) In the inkjet ink according to the present embodiment, the food coloring may have a content in the range of 2.5 mass% or more and 10 mass% or less of the total amount of the inkjet ink, and the ethanol may have a content in the range of 12 mass% or more and 55 mass% or less of the total amount of the inkjet ink, and the surfactant having an HLB value in the range of 16 or more and 20 or less may have a content in the range of 0.5 mass% or more and 5 mass% or less of the total amount of the inkjet ink.

Such a configuration further reduces ink ejection failure while reliably providing excellent transfer durability compared to conventional technologies.

(4) In the inkjet ink according to the present embodiment, the food coloring may have a content in the range of 2.5 mass% or more and 10 mass% or less of the total amount of the inkjet ink, and the ethanol may have a content in the range of 12 mass% or more and 55 mass% or less of the total amount of the inkjet ink, and the sucrose fatty acid ester have a content in the range of 0.5 mass% or more and 5 mass% or less of the total amount of the inkjet ink.

Such a configuration further reduces ink ejection failure while reliably providing excellent transfer durability compared to conventional technologies.

(5) The food coloring contained in the inkjet ink according to the present embodiment may be sodium copper chlorophyllin.

Such a configuration improves transfer durability and lightfastness compared to conventional technologies.

(6) The tablet of the present embodiment is provided with a printed image 3 which has been printed using the inkjet ink described above.

Such a configuration imparts edible properties to the printed image portion printed on the surface of the tablet.

(7) The tablet according to the present embodiment may be a medical tablet.

Such a configuration imparts edible properties to the printed image portion printed on the surface of the medical tablet.

### [Examples]

The present invention will be described in more detail by way of examples; however, the present invention is not limited to the examples.

### (Production of inkjet ink)

A procedure of preparing an inkjet ink will be described.

An inkjet ink contains components, specifically, food coloring, an organic solvent, water, and a surfactant. The first step of the preparation was to mix water with an organic solvent to produce a mixed solvent. Next, a surfactant was then added to the mixed solvent to produce a transparent base liquid. Finally, food coloring was added to the transparent base liquid. In this way, the ink according to the present example was prepared. Each component will be specifically described below.

As water composing the ink, purified water (ion-exchanged water) was used.

Ethanol was used as the organic solvent.

Sucrose fatty acid ester (DK ester SS manufactured by DKS Co. Ltd.), decaglyceryl monostearate (MSW-7S, manufactured by Sakamoto Yakuhin Kogyo Co., Ltd.), and decaglyceryl monocaprylate (MCA-750, manufactured by Sakamoto Yakuhin Kogyo Co., Ltd.) were used as the surfactant.

In each of the present examples, the above components were stirred and mixed to prepare a clear base liquid. After that, various pigments were mixed to obtain inkjet inks of Examples 1 to 29 and Comparative Examples 1 and 2. The composition of each inkjet ink thus obtained is shown in Tables 1 and 2. In Tables 1 and 2, a blank indicates that the substance is not used.

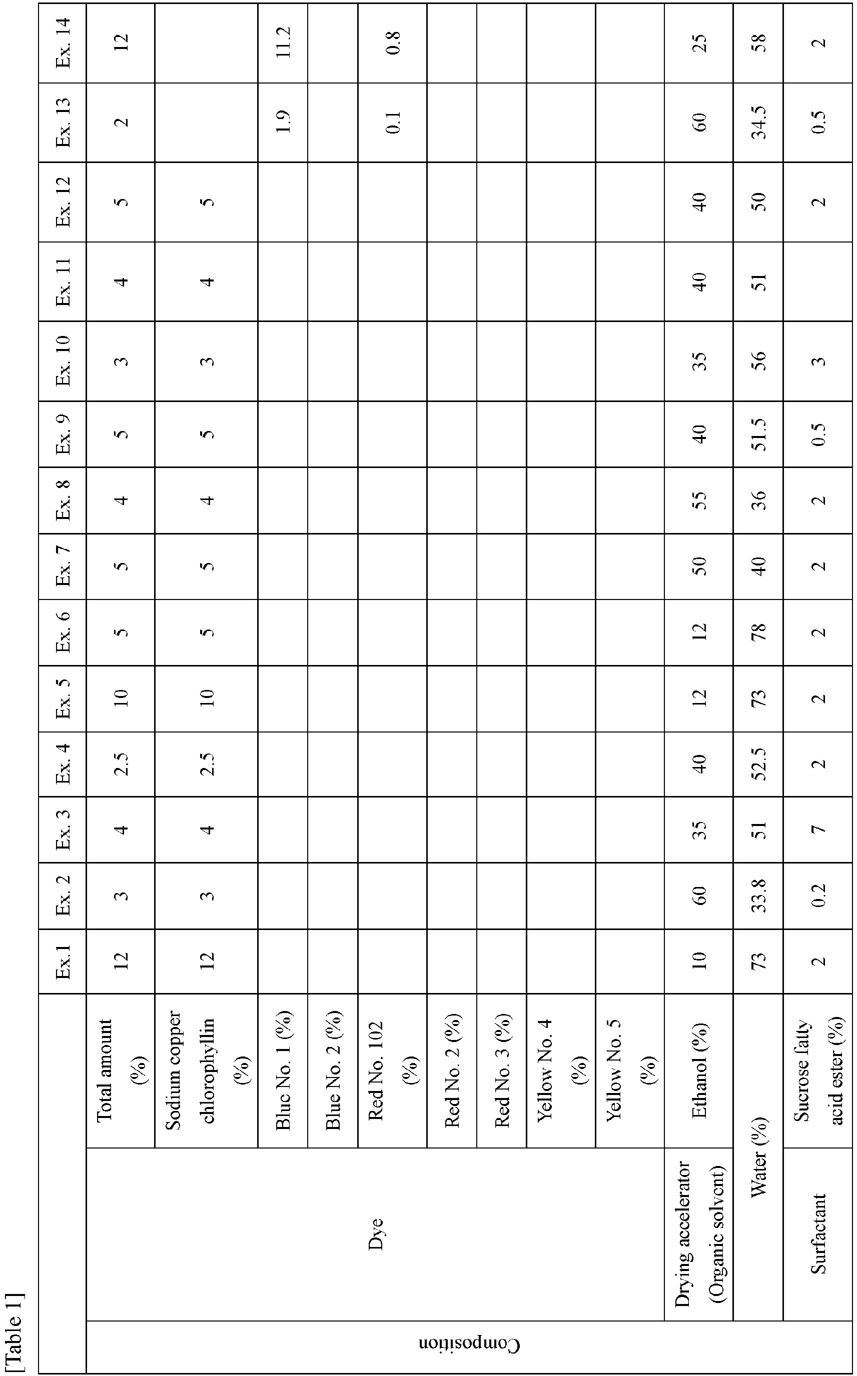

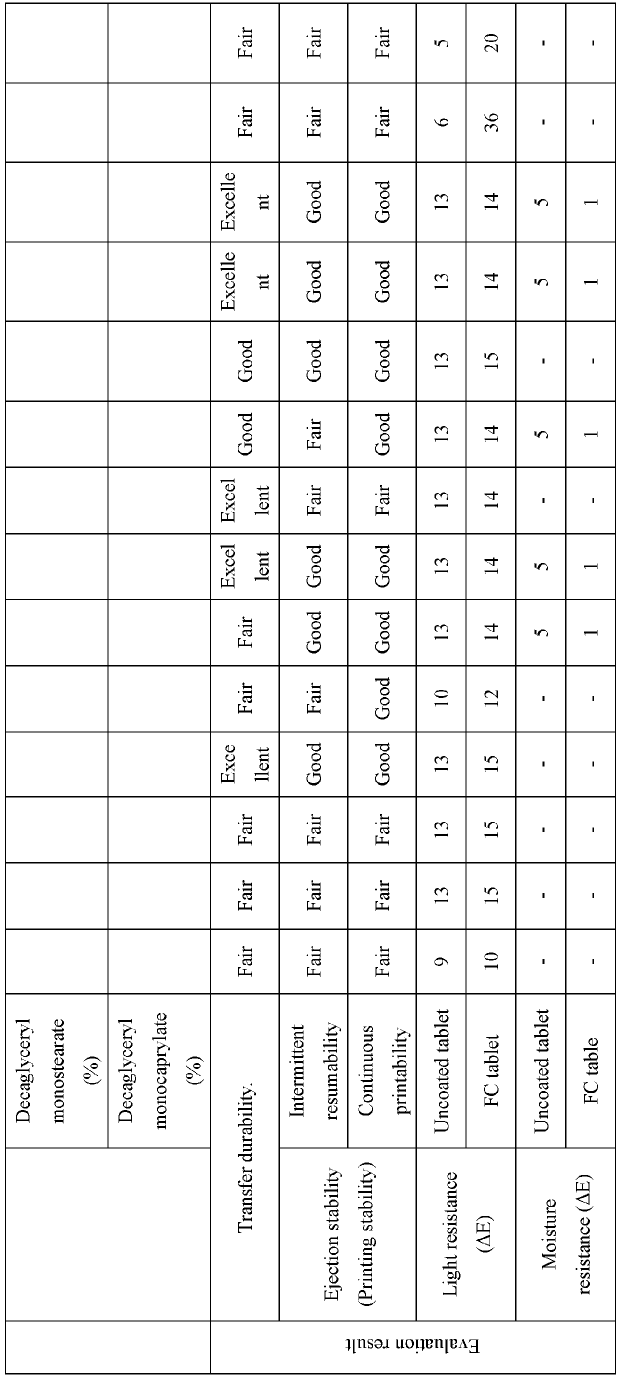

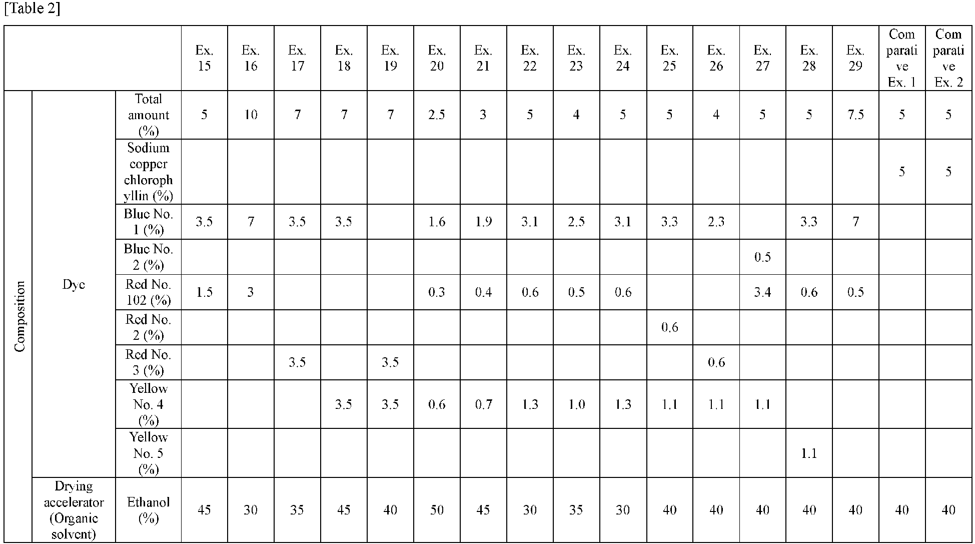

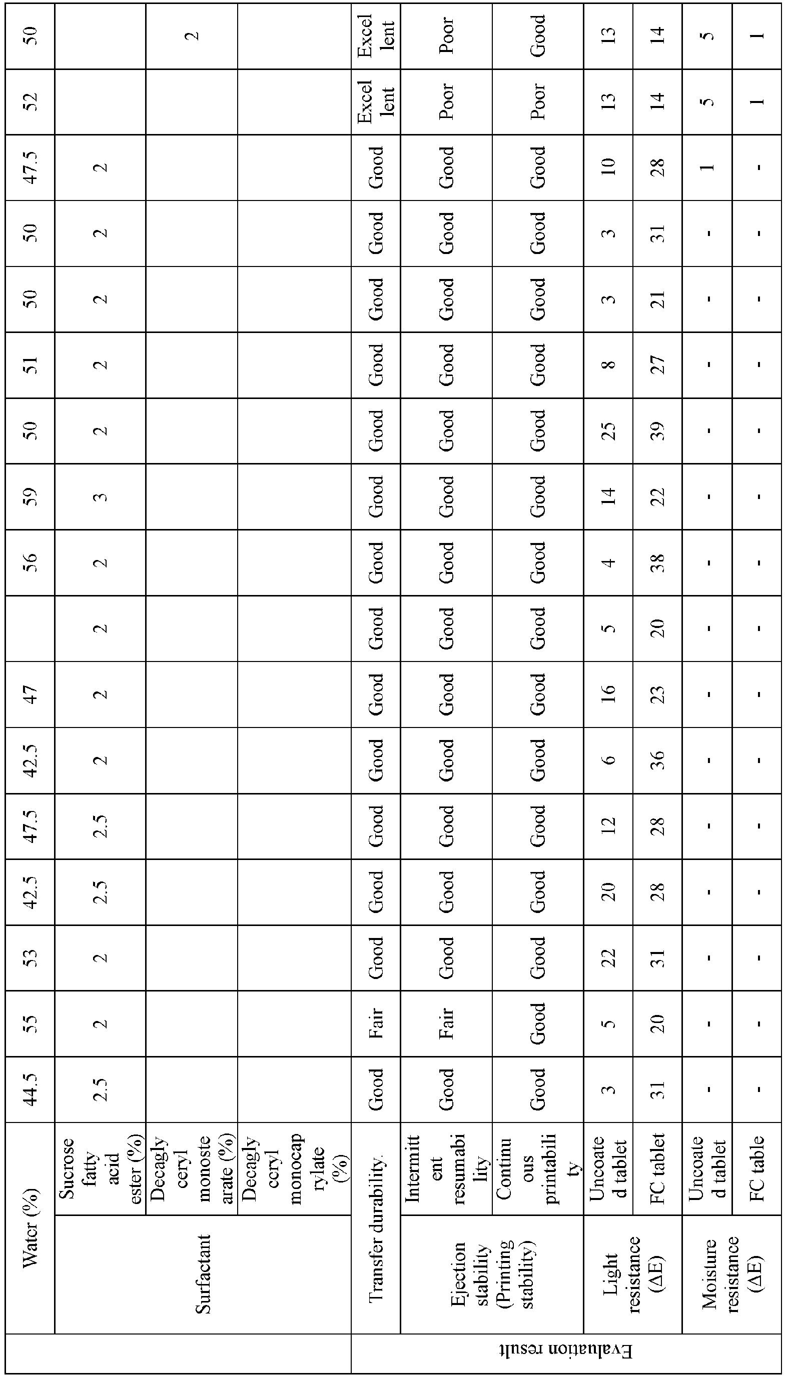

Solid foreign matter in the liquid was removed by passing each above-mentioned 100 grams of ink through a membrane filter. In detail, each ink was passed once through a membrane filter (cellulose acetate film) having a pore diameter of 5.0 µm. Subsequently, each ink was passed once through a membrane filter (cellulose acetate film) having a pore diameter of 0.8 µm to thereby obtain purified ink, each of which weighed 99 g.

### (Transfer durability)

A piezoelectric ceramic-driven drop-on-demand inkjet head having a printing resolution of 600 dpi in a main scanning direction, 600 dpi in a sub scanning direction (conveyance direction of a recording medium such as a tablet), and having 2,656 nozzles in total was used as the inkjet head. Using this inkjet head, a tablet was printed using each ink for 10 seconds at a rate of 10 pl, 6 pl, and less than 6 pl per drop, and then a tablet attached to a digital force gauge was brought into contact with the printed tablet at a pressure of 2 to 3N for 0.4 to 0.5 seconds, and it was confirmed whether the ink did not transfer. In Tables 1 and 2, as a result of evaluating the transfer durability, the presence/absence of transfer for each ejection amount was visually evaluated. The evaluation criteria were as follows.
Excellent: No transfer confirmed at ejection amount of 10 pl
Good: No transfer confirmed at ejection amount of 6 pl
Fair: No transfer confirmed at ejection amount of less than 6 pl
Poor: Transfer confirmed at ejection amount of less than 6 pl

As long as the transfer durability is evaluated as being "Excellent", "Good", or "Fair", the ink has high quality. Even if the transfer durability is evaluated as being "Poor", the ink has no problem in terms of quality (drug efficacy or the like), although visibility remains a problem.

### (Ejection stability)

### <Continuous printability>

A piezoelectric ceramic-driven drop-on-demand inkjet head having a printing resolution of 600 dpi in a main scanning direction, 600 dpi in a sub scanning direction (conveyance direction of a recording medium such as a tablet), and having 2,656 nozzles in total was used as the inkjet head. Using this inkjet head, a test pattern was continuously printed using each ink for 30 minutes at a rate of 6 pl per drop, and after the lapse of 30 minutes, it was confirmed whether the ink had been ejected from all the nozzles without causing ejection failure.

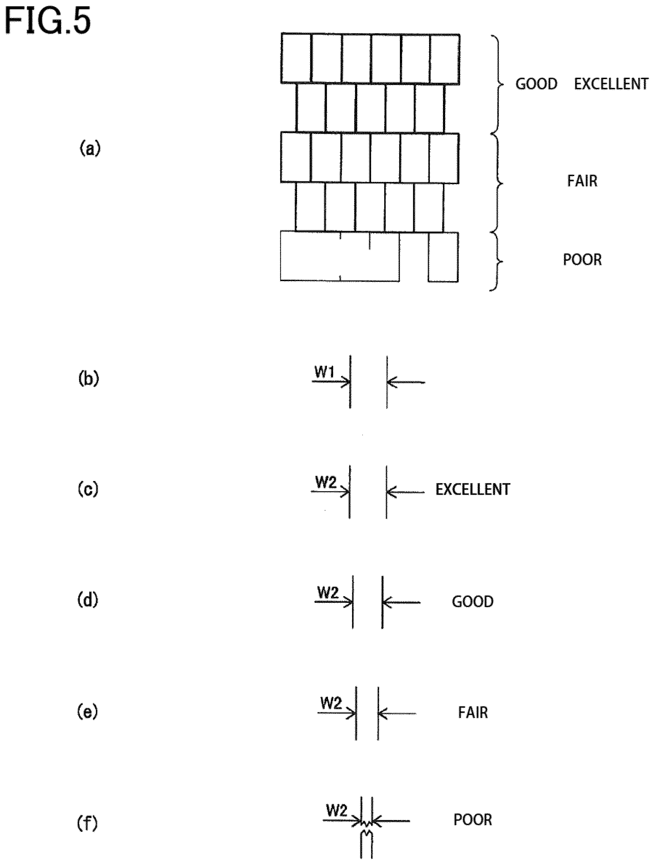

Referring to Figs. 5(a) to 5(f), evaluation criteria for continuous printability according to the present example will be described.

Fig. 5 is a set of diagrams illustrating evaluation criteria for evaluating printing stability (continuous printability) in the present example. Fig. 5(a) shows a test pattern used to evaluate the continuous printability in the present example. This test pattern is configured by straight lines extending in the horizontal direction of Fig. 5(a) and straight lines extending in the vertical direction of Fig. 5(a), and each straight line has a width of 0.5 mm. Fig. 5(b) shows a line width W1 immediately after the start of printing of the test pattern. Figs. 5(c) to 5(f) each show a line width W2 immediately after the lapse of 30-minute continuous printing of the test pattern. As shown in Fig. 5(c), in the present example, ink was evaluated as being "Excellent", if the line width W2 immediately after the lapse of 30-minute continuous printing of the test pattern was not decreased compared to the line width W1 immediately after the start of printing of the test pattern, i.e., if the decrease ratio of the line width was substantially 0%. As shown in Fig. 5(d), in the present example, ink was evaluated as being "Good", if the line width W2 immediately after the lapse of 30-minute continuous printing of the test pattern was decreased compared to the line width W1 immediately after the start of printing of the test pattern, i.e., if the decrease ratio of the line width was 10% or less. As shown in Fig. 5(e), in the present example, ink was evaluated as being "Fair", if the line width W2 immediately after the lapse of 30-minute continuous printing of the test pattern was decreased compared to the line width W1 immediately after the start of printing of the test pattern, i.e., if the decrease ratio of the line width exceeded 10%, but the print lines configuring the test pattern had no line breakages (no disconnected portions or unprinted portions). As shown in Fig. 5(f), in the present example, ink was evaluated as being "Poor", if there were line breakages (unprinted portions) in the print lines immediately after the lapse of 30-minute continuous printing of the test pattern.

The evaluation criteria for continuous printability in the above present examples are summarized as follows.

Excellent: Ejection failure was not observed at any of the nozzles. In addition, no decrease in line width was visually observed in the test pattern.

Good: Ejection failure was not observed at any of the nozzles, but decrease in line width (10% or less) was visually observed.

Fair: Ejection failure was not observed at any of the nozzles, but decrease in the ink ejection amount was observed in some nozzles. Accordingly, decrease in line width (exceeding 10%) was visually observed in the test pattern. However, no line breakages (unprinted portions) were observed in the test pattern.

Poor: Ejection failure was observed in some nozzles. Accordingly, line breakages (unprinted portions) were observed in the test pattern.

As long as the evaluation for the continuous printability is "Excellent", "Good", or "Fair", the ink has high quality. Even if the continuous printability is evaluated as "Poor", the ink has no problem in terms of quality (drug efficacy or the like), although visibility remains a problem.

### intermittent resumability (print resumability)>

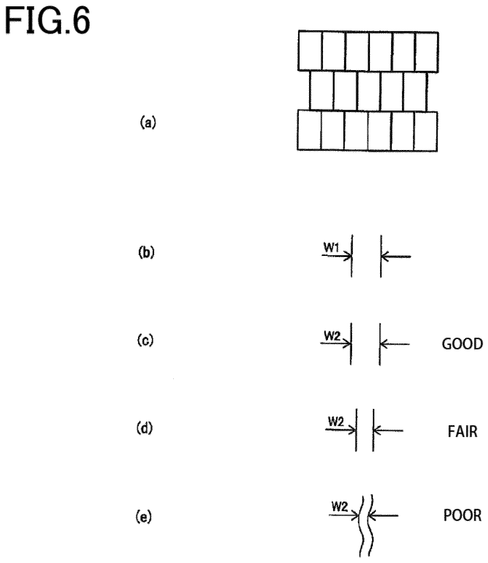

Referring to Figs. 6(a) to 6(e), evaluation criteria for intermittent resumability according to the present example will be described.

Fig. 6 is a set of diagrams illustrating evaluation criteria for evaluating ejection stability (intermittent resumability) in the present example. Fig. 6(a) shows a test pattern used to evaluate the intermittent resumability in the present example. The test pattern is composed of a straight line extending in the horizontal direction in Fig. 6 (a) and a straight line extending in the vertical direction in Fig. 6 (a), and the line width of each straight line is 0.5 mm. Fig. 6(b) shows a test pattern line width W1 immediately after ink injection. Fig. 6(c) shows a line width W2 immediately after printing is resumed following a 30-minute suspension. As shown in Fig. 6(c), in the present example, if the line width W2 immediately after printing is resumed following the 30-minute suspension was not decreased compared with the line width W1 immediately after the ink is injected, or if the line width W2 was decreased by 10% or less compared with the line width W1, the ink is evaluated as "Good".

As shown in Fig. 6(d), the line width W2 immediately after printing is resumed following the 30-minute suspension was decreased compared with the line width W1 immediately after the ink is injected. If the line width W2 was decreased by less than 50%, the ink is evaluated as "Fair". As shown in Fig. 6(e), the line width W2 immediately after printing is resumed following the 30-minute suspension was decreased compared with the line width W1 immediately after the ink is injected. If the line width W2 was decreased by less than 50%, the ink is evaluated as "Poor".

The evaluation criteria for intermittent resumability in the above examples are summarized as follows.

Good: Ejection failure was not observed at any of the nozzles, but decrease in line width (10% or less) was visually observed.

Fair: Ejection failure was not observed at any of the nozzles, but decrease in the ink ejection amount was observed in some nozzles. Accordingly, decrease in line width (50% or less) was visually observed in the test pattern. However, no line breakages (unprinted portions) were observed in the test pattern.

Poor: Ejection failure was observed in some nozzles. Accordingly, line breakages (unprinted portions) were observed in the test pattern.

As long as the evaluation for the intermittent resumability is "Excellent", "Good", or "Fair", the ink has high quality. Even if the intermittent resumability is evaluated as "Poor", the ink has no problem in quality (drug efficacy or the like), although visibility remains a problem.

### (Light fastness)

The purified ink was used to print a circular solid image (4.0 mm in diameter) with a bending mode piezo inkjet head printer on a test uncoated tablet (based on adjusted starch) and a film-coated tablet (based on adjusted starch, coated with hydroxypropyl methylcellulose 70%, 30% mixture with titanium oxide, 6.5 mm in diameter). From this result, a printed uncoated tablet and a printed film-coated tablet were obtained.

The uncoated tablet and the film-coated tablet whose chromaticity and optical color density were measured were irradiated with visible light of cumulative 1.2 million lux using a xenon weather meter (Ci 4000, Toyo Seiki Seisaku-sho, Ltd.). Chromaticity and optical color density were measured with a spectrophotometer on the uncoated tablet and the film-coated tablet irradiated with visible light, and the amount of change (ΔE) of chromaticity and optical color density before and after the test was compared. The results of comparison are shown in Tables 1 and 2.

An opinion test conducted by the inventors together with various medical personnel regarding the change in color after the moisture resistance test led to the conclusion that if ΔE≤15, the ink has sufficient lightfastness as an inkjet ink for medical tablets. Therefore, ΔE ≤ 15 was considered acceptable for this evaluation.

Here, as shown in Tables 1 and 2, in the film-coated tablet of Example 13 to 29, ΔE is 16 or more, which is insufficient for evaluation of light resistance, but sufficient for each evaluation of transfer durability and ejection stability.

### (Moisture resistance)

An uncoated tablet and a film-coated tablet for which chromaticity and optical color density were measured were placed in a thermostatic humidistat at 40°C and 90% humidity, and after 24 hours, chromaticity and optical color density were measured for the uncoated tablet and the film-coated tablet after the moisture resistance test using the thermostatic humidistat, and the amount of change (ΔE) of chromaticity and optical color density before and after the test were compared. The results of comparison are shown in Tables 1 and 2.

An opinion test conducted by the inventors together with various medical personnel regarding the change in color after the moisture resistance test led to the conclusion that if ΔE≤15, the ink has sufficient moisture resistance as an inkjet ink for medical tablets. Therefore, ΔE ≤ 15 was considered acceptable for this evaluation.

In Tables 1 and 2, "-" indicates that no moisture resistance test has been performed.

The above results show that an inkjet ink containing food coloring, ethanol, and a surfactant having an HLB value in the range of 16 or more and 20 or less, specifically sucrose fatty acid ester (DK ester SS manufactured by DKS Co. Ltd.), can obtain high ejection stability while maintaining excellent transfer durability even when the inkjet ink is stored for a long time or continuous printing is performed using the inkjet ink. The food coloring is such as sodium copper chlorophyllin, Blue No. 1, Blue No. 2, Red No. 102, Red No. 2, Red No. 3, Yellow No. 4, or Yellow No. 5. More specifically, using sucrose fatty acid ester as the surfactant improves the ejection stability. Also, even when the kind of food coloring is changed, an effect on ejection stability can be obtained. On the other hand, when the sucrose fatty acid ester was not added or when decaglyceryl monostearate, which is a type of glycerin fatty acid ester that has been commonly used as a discharge stabilizer, was added, the effect of discharge stability confirmed in the present embodiment could not be obtained.

The inkjet ink of this example contains a relatively large amount of volatile alcohol, and therefore has excellent transfer durability.

### [Reference Signs List]

1: Tablet
3: Printed image
5: Uncoated tablet
7: Film coating layer
9: Film-coated tablet
11: Printed image on uncoated tablet (solid image)
13: Printed image on film coated tablet (two-dimensional barcode)

## Claims

1. An edible aqueous inkjet ink comprising:
a dye;
ethanol; and
a surfactant which has a hydrophilic-lipophilic balance value in a range of 16 or more and 20 or less.

2. An edible aqueous inkjet ink comprising:
a dye;
ethanol; and
sucrose fatty acid ester.

3. The edible aqueous inkjet ink according to claim 1, wherein
the dye has a content in a range of 2.5 mass% or more and 10 mass% or less of a total amount of the edible aqueous inkjet ink, and wherein
the ethanol has a content in a range of 12 mass% or more and 55 mass% or less of the total amount of the edible aqueous inkjet ink, and wherein
the surfactant has a content in a range of 0.5 mass% or more and 5 mass% or less of the total amount of the edible aqueous inkjet ink.

4. The edible aqueous inkjet ink according to claim 2, wherein
the dye has a content in a range of 2.5 mass% or more and 10 mass% or less of a total amount of the edible aqueous inkjet ink, and wherein
the ethanol has a content of in a range of 12 mass% or more and 55 mass% or less of the total amount of the edible aqueous inkjet ink, and wherein
the sucrose fatty acid ester has a content in a range of 0.5 mass% or more and 5 mass% or less of the total amount of the edible inkjet ink.

5. The edible aqueous inkjet ink according to any one of claims 1 to 4, wherein the dye is sodium copper chlorophyllin.

6. A tablet comprising a portion which is printed with the edible aqueous inkjet ink according to any one of claims 1 to 5.

7. The tablet according to claim 6, wherein the tablet is a medical tablet.
